# EUROPÄISCHE PATENTANMELDUNG

(11) **EP 2 853 275 A1**
(43) Veröffentlichungstag der Anmeldung: **01.04.2015**
(21) Anmeldenummer: 13186047.0
(22) Anmeldetag: 25.09.2013
(51) Int. Cl.: A61L 2/20, B65D 88/12

(54) **Stationäre Ethylenoxid-Sterilisationsanlage**

(71) Anmelder: B. Braun Melsungen AG, 34212 Melsungen (DE)
(72) Erfinder: GEIGER, Ralph, 34587 Felsberg (DE)
(74) Vertreter: Arth, Hans-Lothar

(57) **Zusammenfassung**

Die vorliegende Erfindung betrifft eine stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, wobei sich die Sterilisationskammer, die Vakuumpumpe, der Ethylenoxidverdampfer und der Ethylenoxidtank in mindestens einem Metallcontainer befinden.

## Beschreibung

Die vorliegende Erfindung betrifft eine stationäre Ethylenoxid-Sterilisationsanlage umfassend eine Vakuumpumpe, einen Ethylenoxidverdampfer, einen Ethylenoxidtank oder Ethylenoxidflasche, eine Steuerung, eine Befeuchtungsanlage (Dampfgenerator) und eine Sterilisationskammer, wobei sich die Sterilisationskammer, die Vakuumpumpe, der Ethylenoxidverdampfer und der Ethylenoxidtank in einem Metallcontainer oder mehreren Metallcontainern befinden. Vakuumpumpe, Ethylenoxidverdampfer und Ethylenoxidtank befinden sich in einem Metallcontainer, wobei sich die Sterilisationskammer in demselben oder einem weiteren Metallcontainer befinden kann.

Bestehende industriell genutzte Ethylenoxid(EO)-Sterilisationsanlagen (Palettensterilisationsanlagen) werden vor Ort beim Betreiber endmontiert. Hierbei entsteht ein erheblicher Aufwand während der Installation und Inbetriebnahme. Die Versorgungseinheiten sind in verschiedenen Räumen untergebracht und werden über Rohrleitungen miteinander verbunden.

Zusätzlich muss für die einzelnen Aufstellungsräume der Explosionsschutz gebäudeseitig mit beachtet werden. Es ergibt sich daraus zusätzlich ein erhöhter Aufwand für das EO-Monitoring, sowie für die Gebäudelüftung (explosionssichere Auslegung sowie ein stetiger Luftaustausch im Normalbetrieb in den Räumen, sowie erhöhte Notlüftung bei Leckage).

Der Erfindung liegt somit die Aufgabe zugrunde, eine stationäre Ethylenoxid-Sterilisationsanlage bereitzustellen, welche sicher ist aber die Installationskosten deutlich reduziert.

Es wurde gefunden, dass diese Aufgabe gelöst wird, indem die explosionsgefährdeten Komponenten einer stationären Ethylenoxid-Sterilisationsanlage in einem Metallcontainer oder mehreren Metallcontainern bzw. vorzugsweise zwei Metallcontainern untergebracht werden.

Somit betrifft die vorliegende Erfindung eine stationäre Ethylenoxid-Sterilisationsanlage umfassend eine Vakuumpumpe, einen Ethylenoxidverdampfer, einen Ethylenoxidtank, eine Steuerung, eine Befeuchtungsanlage (Dampfgenerator) und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind.

Unter dem Begriff "mindestens einem Metallcontainer" soll verstanden werden, dass sich die vorgenannten Komponenten in einem Metallcontainer befinden sich aber auch auf mehrere Metallcontainer und vorzugsweise auf zwei Metallcontainer verteilen können.

Somit verteilen sich die explosionsgefährdeten Komponenten der stationären Ethylenoxid-Sterilisationsanlage nicht über verschiedene Räume, sondern befinden sich zusammen in einem oder mehreren Metallcontainern . Bei den explosionsgefährdeten Komponenten der stationären Ethylenoxid-Sterilisationsanlage handelt es sich um die Sterilisationskammer, die Vakuumpumpe, den Ethylenoxidverdampfer und den Ethylenoxidtank.

Bei dem Metallcontainer oder den Metallcontainern handelt es sich vorzugsweise um Seecontainer, d.h. normierte 20 ft (6,096 m) oder 40 ft (12,192) Container. Diese Seecontainer haben den großen Vorteil der Normierung, so dass die nach Installation am Bestimmungsort stationäre Ethylenoxid-Sterilisationsanlage in den normierten Seecontainern vollständig eingebaut werden und in diesem Zustand per LKW, Zug oder Schiff mit geringem Aufwand transportiert werden kann. Am Bestimmungsort angekommen, brauchen dann die normierten Container nur noch mit den Außenleitungen verbunden zu werden, ohne dass die Einzelkomponenten der stationären Ethylenoxid-Sterilisationsanlage jeweils einer Installation bedürfen. Dies ist vor allem ein enormer Vorteil, wenn derartige stationäre Ethylenoxid-Sterilisationsanlagen in Schwellenländer wie beispielsweise Indien, Südamerika oder einigen asiatischen Staaten wie z.B. Indonesien, Malaysia, Philippinen, Vietnam, Thailand oder der gleichen geliefert werden, wo jede Installation vor Ort die Anwesenheit ausländischer Mechaniker und Techniker bedarf.

Unter dem Begriff "stationäre" Ethylenoxid-Sterilisationsanlage soll verstanden werden, dass nach Installation der Ethylenoxid-Sterilisationsanlage am Bestimmungsort diese Anlage zu einer stationären Anlage wird, d.h. dauerhaft am Bestimmungsort verbleibt und nur durch aufwändige Demontage und erneute Installation an einen anderen Ort zu bringen ist. Dieser Begriff "stationär" schießt natürlich nicht aus, dass vor der Endinstallation am Bestimmungsort die in dem mindestens einen Container und vorzugsweise mindestens einen Seecontainer montierte Ethylenoxid-Sterilisationsanlage transportiert werden kann. Genau in dieser Kombination der Merkmale liegt der große Vorteil der erfindungsgemäßen stationären Ethylenoxid-Sterilisationsanlage. Zum einen ist die Ethylenoxid-Sterilisationsanlage bis auf die Außenanschlüsse in dem mindestens einen Container und vorzugsweise in dem mindestens einen Seecontainer fast vollständig vormontiert und zum anderen befindet sich die erfindungsgemäße Ethylenoxid-Sterilisationsanlage in einem Gehäuse, nämlich mindestens einem Metallcontainer und vorzugsweise mindestens einem Seecontainer, so dass ein Transport auch über weite Strecken unmittelbar, einfach und kostengünstig erfolgen kann. Am Bestimmungsort angekommen, muss dann die stationäre Ethylenoxid-Sterilisationsanlage nicht noch aufwändig zusammengebaut werden, sondern braucht nur noch mit den Rohrleitungen von außen, d.h. den Außenanschlüssen verbunden zu werden und ist betriebsbereit. Somit ist die Vorrichtung für die Auslieferung der stationären Ethylenoxid-Sterilisationsanlage identisch mit dem Gehäuse für die stationäre Ethylenoxid-Sterilisationsanlage, weil der Metallcontainer und vorzugsweise der normierte Seecontainer beide Aufgaben übernimmt.

Daher ist eine bevorzugte Ausführungsform der vorliegenden Erfindung, dass sich zumindest Sterilisationskammer, Vakuumpumpe, Ethylenoxidverdampfer und Ethylenoxidtank in einem normierten Metallcontainer und vorzugsweise einen normierten Seecontainer befinden.

Für den Fall dass eine möglichst große Sterilisationskammer gewünscht wird, betrifft eine weitere bevorzugte Ausführungsform der vorliegenden Erfindung eine stationäre Ethylenoxid-Sterilisationsanlage, welche in zwei normierten Metallcontainern und vorzugsweise zwei normierten Seecontainern untergebracht ist. Bei dieser Ausführungsform wird die Sterilisationskammer in einem Metallcontainer untergebracht, so dass der maximale Raum, nämlich der Raum eines Metallcontainers und vorzugsweise eines normierten Seecontainers zur Verfügung steht und in einem zweiten Metallcontainer zumindest Vakuumpumpe, Ethylenoxidverdampfer und Ethylenoxidtank vorgesehen werden.

Sollte damit die Größe der Sterilisationskammer noch immer nicht ausreichen, so kann in einer weiteren bevorzugten Ausführungsform der erfindungsgemäßen stationären Ethylenoxid-Sterilisationsanlage vorgesehen werden, dass zumindest Vakuumpumpe, Ethylenoxidverdampfer und Ethylenoxidtank in einem ersten normierten Metallcontainer untergebracht sind und an diesen ersten Metallcontainer zwei oder mehr normierte Metallcontainer angeschlossen werden können, worin jeweils Sterilisationskammern vorgesehen sind, so dass der für die Sterilisation zur Verfügung stehende Raum durch einen dritten Metallcontainer verdoppelt, durch einen vierten verdreifacht und durch einen (X-1)-Metallcontainer X-fach vergrößert werden kann.

Da somit innerhalb des Metallcontainers bzw. der Metallcontainer der explosionsgefährdete Raum ist, erfolgt die Steuerung der stationären Ethylenoxid-Sterilisationsanlage vorzugsweise von außerhalb des Metallcontainers. Die Steuerung oder Steuervorrichtung für die stationäre Ethylenoxid-Sterilisationsanlage ist daher vorzugsweise außerhalb des mindestens einen Metallcontainers oder von außen am Metallcontainer angebracht. Ist die Steuerung hingegen im Metallcontainer untergebracht, so befindet sich diese vorzugsweise in einem innerhalb des Metallcontainers räumlich abgetrennten Bereich. Werden zwei oder mehr normierte Metallcontainer verwendet, so befinden sich im ersten normierten Metallcontainer zumindest Vakuumpumpe, Ethylenoxidverdampfer und Ethylenoxidtank und optional Befeuchtungsanlage, Abgasreinigungsanlage und Kammerheizung. In dem oder den weiteren normierten Metallcontainers befinden sich Sterilisationskammern. Auch vorzugsweise außerhalb des Metallcontainers oder der Metallcontainer befindet sich die EO-Entsorgung.

Es ist vorteilhaft, wenn sich an der Decke des Metallcontainers oder der Metallcontainer eine Druckentlastungsbereich befindet.

Die Unterbringung der explosionsgefährdeten Bauteile der stationären Ethylenoxid-Sterilisationsanlage in einem Metallcontainer hat ferner den Vorteil, dass die Anlage in diesem Metallcontainer vormontiert werden kann, der komplette Metallcontainer vom Hersteller zum Kunden ausgeliefert und dort die Endmontage deutlich einfacher vorgenommen werden kann, weil nur noch die entsprechenden Zu- und Ableitungen zu dem oder den Metallcontainer(n) geschaffen werden müssen aber die Sterilisationsanlage an sich bereits fertig im Metallcontainer montiert ist. Damit wird die Installation vor Ort vereinfacht. Zusätzliche Verrohrungsarbeiten zwischen den explosionsgefährdeten Bauteilen der Sterilisationsanlage vor Ort sind nicht mehr notwendig. Zudem werden die prozessführenden Rohrleitungen kürzer, da sich alle relevanten Bauteile in mindestens einem Metallcontainer nahe beieinander befinden und nicht mehr räumlich getrennt sind. Um die Installation vor Ort so einfach wie möglich zu gestalten, können auch die Steuerung für die EO-Sterilisationsanlage und die Befeuchtungsanlage innerhalb des Metallcontainers angeordnet sein, worin sich Vakuumpumpe, Ethylenoxidverdampfer und Ethylenoxidtank befinden und vorzugsweise durch eine räumliche Abtrennung von den anderen Komponenten der EO-Sterilisationsanlage separiert werden.

Ein weiterer Vorteil liegt darin, dass Störfälle (insbesondere Leckagen) durch den mindestens einen Metallcontainer räumlich begrenzt werden. Durch die vorzugsweise außen am Metallcontainer angebrachte Steuerung, welche den Zustand der Sterilisationsanlage samt der explosionsgefährdeten Bauteile anzeigt, wird die Mitarbeitergefährdung minimiert. Ein Zugang zum mindestens einen Metallcontainer wird beispielsweise nur erlaubt, wenn keine unmittelbare Gefahr besteht. Die Anordnung der explosionsgefährdeten Bauteile der stationären Ethylenoxid-Sterilisationsanlage hat zudem den Vorteil, dass im laufenden Betrieb eine Überwachung von mehreren Räumen (EO-Waagenraum, EO-Verdampferraum, Sterilisationsbereich) nicht mehr notwendig ist. Die Überwachung des Containerinnenraums oder der Containerinnenräume bzw. der Abluft dieser Räume genügt vollkommen.

### Figur 1

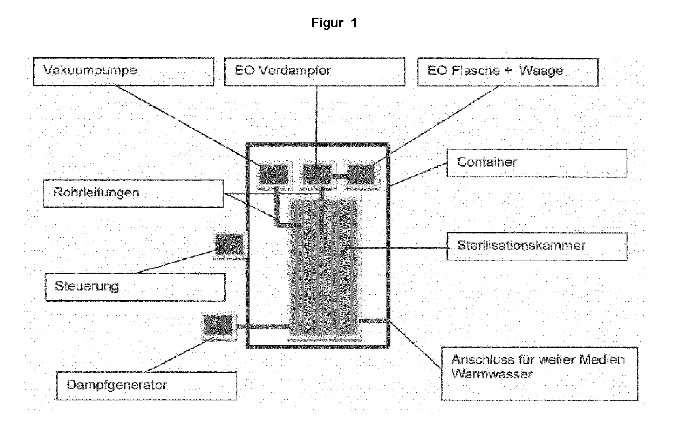

Figur 1 zeigt einen schematischen Aufbau einer erfindungsgemäßen stationären Ethylenoxid-Sterilisationsanlage umfassend eine Vakuumpumpe 5, einen Ethylenoxidverdampfer 4, einen Ethylenoxidtank 3, eine Steuerung oder Steuervorrichtung 6, eine Befeuchtungsanlage 7 in Form eines Dampfgenerators 7, erforderliche Rohrleitungen 8 und eine Sterilisationskammer 2. Die explosionsgefährdeten Bauteile umfassend die Sterilisationskammer 2, die Vakuumpumpe 5, den Ethylenoxidverdampfer 4 und den Ethylenoxidtank 3 sind in dem Metallcontainer 1 bzw. Seecontainer 1 untergebracht. Steuerung oder Steuervorrichtung 6 für die Sterilisationsanlage sowie Befeuchtungsanlage 7 sind außerhalb des Metallcontainers vorgesehen.

Zwingend mittels Rohrleitungen 8 innerhalb des Metallcontainers 1 verbunden sind der Ethylenoxidtank 3 mit dem Ethylenoxidverdampfer 4 und dieser wiederum mit der Sterilisationskammer 2. Die Sterilisationskammer 2 ist des weiteren über Rohrleitungen 8 innerhalb des Metallcontainers 1 mit mindestens einer Vakuumpumpe 5 verbunden. Wie in Figur 1 gezeigt, kann sich der mindestens eine Dampfgenerator 7 bzw. die mindestens eine Befeuchtungsanlage außerhalb des Metallcontainers 1 befinden, kann aber auch innerhalb des Metallcontainers 1 angeordnet sein. Auch zwischen Befeuchtungsanlage 7 und Sterilisationskammer 2 verlaufen entsprechende Rohrleitungen. Die Befeuchtungsanlage ist vorzugsweise ein Dampfgenerator 7. Befindet sich der Dampfgenerator 7 außerhalb des Metallcontainers 1, so führt die Rohrleitung zwischen Dampfgenerator 7 und Sterilisationskammer 2 durch den Containermantel hindurch. Des weiteren gibt es eine Leitung für die Kammerheizung, diese kann sowohl in dem Metallcontainer, als auch außerhalb angeordnet sein. Ist diese außerhalb angeordnet, ist die Rohrleitung zwischen Sterilisationskammer 2 und Warmwassererzeuger eine zweite Rohrleitung die durch den Containermantel hindurchführt. Als Dritte Leitung, welche durch den Containermantel hindurchführt, ist die Leitung von Vakuumpumpe 5 zur Abgasreinigungsanlage. Die Abgasreinigungsanlage ist vorzugsweise außerhalb des Gebäudes angeordnet, kann aber auch innerhalb des Metallcontainers angeordnet werden.

### Figur 4

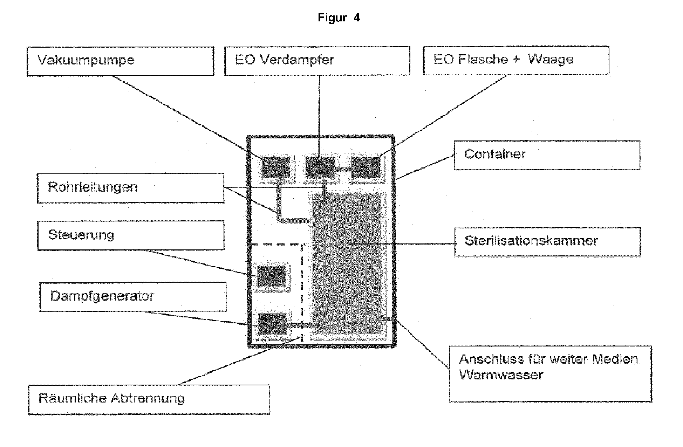

Figur 4 zeigt einen schematischen Aufbau einer erfindungsgemäßen stationären Ethylenoxid-Sterilisationsanlage umfassend eine Vakuumpumpe 5, einen Ethylenoxidverdampfer 4, einen Ethylenoxidtank 3, eine Steuerung oder Steuervorrichtung 6, eine Befeuchtungsanlage 7 in Form eines Dampfgenerators 7, erforderliche Rohrleitungen 8 und eine Sterilisationskammer 2. Die explosionsgefährdeten Bauteile umfassend die Sterilisationskammer 2, die Vakuumpumpe 5, den Ethylenoxidverdampfer 4 und den Ethylenoxidtank 3 sind in dem Metallcontainer 1 bzw. Seecontainer 1 untergebracht. Steuerung oder Steuervorrichtung 6 für die Sterilisationsanlage sowie Befeuchtungsanlage 7 sind ebenfalls innerhalb des Metallcontainers vorgesehen und vorzugsweise in einem räumlich getrennten Bereich innerhalb des Metallcontainers.

Zwingend mittels Rohrleitungen 8 innerhalb des Metallcontainers 1 verbunden sind der Ethylenoxidtank 3 mit dem Ethylenoxidverdampfer 4 und dieser wiederum mit der Sterilisationskammer 2. Die Sterilisationskammer 2 ist des weiteren über Rohrleitungen 8 innerhalb des Metallcontainers 1 mit mindestens einer Vakuumpumpe 5 verbunden. Wie in Figur 4 gezeigt, kann sich der mindestens eine Dampfgenerator 7 bzw. die mindestens eine Befeuchtungsanlage auch innerhalb des Metallcontainers 1 befinden. Auch zwischen Befeuchtungsanlage 7 und Sterilisationskammer 2 verlaufen entsprechende Rohrleitungen innerhalb des Metallcontainers. Die Befeuchtungsanlage ist vorzugsweise ein Dampfgenerator 7. Auch die Kammerheizung ist in dem Metallcontainer untergebracht. Eine Leitung verläuft zwischen Kammerheizung und Sterilisationskammer 2 innerhalb des Metallcontainers. Eine Leitung, welche durch den Containermantel hindurchführt, ist die Leitung von Vakuumpumpe 5 zur Abgasreinigungsanlage. Die Abgasreinigungsanlage ist vorzugsweise außerhalb des Gebäudes angeordnet, kann aber auch innerhalb des Metallcontainers angeordnet werden.

Der Metallcontainer 1 hat vorzugsweise zwei Zugänge, welche zudem bevorzugt gegenüber liegen. Zwei Zugänge sind zum einen für die Beladung der Sterilisationskammer 2 mit den zu sterilisierenden Waren vorteilhaft, weil so die Sterilisationskammer 2 von der einen Seite beladen und von der anderen Seite entladen werden kann. Darüber hinaus ist ein zweiter Zugang für die Einbringung und Auswechslung des Ethylenoxidtanks 3 vorteilhaft. Möglich sind auch Metallcontainer 1 mit mehr als zwei Zugängen, wobei sich vorzugsweise zwei Zugänge gegenüber liegen. Bei derartigen Metallcontainern 1 weist die Sterilisationskammer 2 dann vorzugsweise auch zwei vorzugsweise gegenüberliegende Zugänge auf, so dass die Sterilisationskammer 2 von einer Seite beladen und von der gegenüberliegenden Seite entladen werden kann.

Die stationäre Ethylenoxid-Sterilisationsanlage dient vorzugsweise zur Sterilisation von Medizinprodukten und weiter bevorzugt zur Sterilisation von Medizinprodukten im Palettenverfahren. Eine manuelle Beladung mit einzelnen Medizinprodukten im Karton ist damit grundsätzlich auch möglich, wobei vorzugsweise Paletten verwendet werden.

Somit handelt es sich vorzugsweise bei der stationären Ethylenoxid-Sterilisationsanlage um eine Palettensterilisationsanlage, d.h. eine stationäre Ethylenoxid-Palettensterilisationsanlage.

Der Begriff "stationär" ist dahingehend auszulegen, dass die EO-Sterilisationsanlage nach Installation bzw. Endmontage nicht mehr mobil ist und fest an dem vorgesehenen Platz verbleibt. Zudem stellt der Begriff "stationär" fest, dass es sich nicht um eine tragbare EO-Sterilisationsanlage handelt, sondern um eine großtechnische Anlage, welche für die Sterilisation ganzer Paletten vorgesehen ist.

### Figur 2

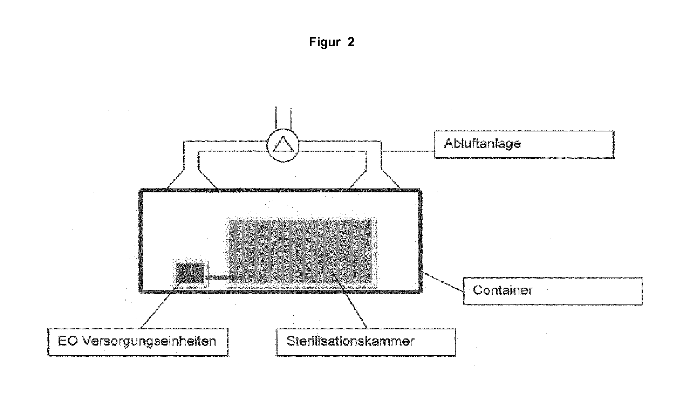

Figur 2 zeigt die Anbindung einer erfindungsgemäßen stationären Ethylenoxid-Sterilisationsanlage an eine Abluftanlage 9.

Zuluftleitungen als auch Abluftleitungen können direkt mit dem Metallcontainer 1 verbunden werden und müssen nicht mehr für jeden einzelnen Raum vorgesehen werden, in dem sich ein Bauteil und insbesondere ein explosionsgefährdetes Bauteil der Sterilisationsanlage befindet. Durch die separate Containerraumluftabführung sind gebäudeseitige explosionsgeschützte Abluftanlagen der verschiedenen Sterilisationsbereiche daher nicht mehr notwendig.

### Figur 3

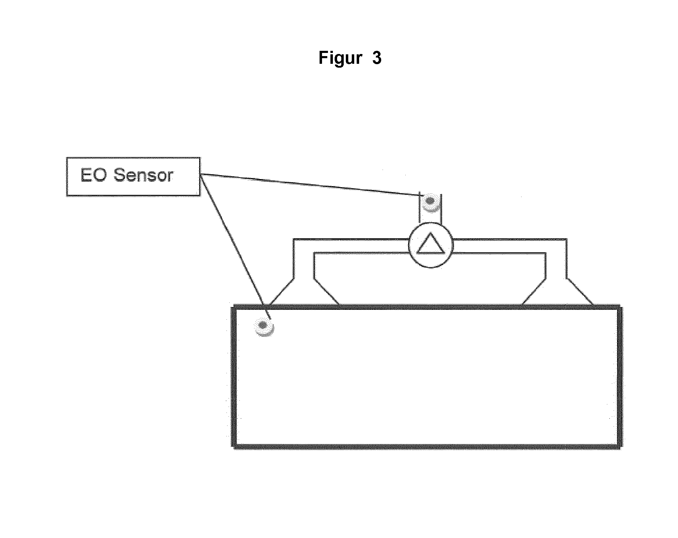

Figur 3 zeigt bevorzugte Stellen, wo EO-Sensoren 10 in der Anbindung der erfindungsgemäßen stationären Ethylenoxid-Sterilisationsanlage an eine Abluftanlage 9 vorgesehen werden können.

Grundsätzlich genügt ein Ethylenoxid(EO)-Sensor 10 in der Abluftleitung 9 oder innerhalb des Metallcontainers 1 (dargestellt durch die beiden Punkte). Vom Metallcontainer 1 ist nur der Umriß wiedergegeben, die Sterilisationsanlage im Inneren des Metallcontainers 1 ist in Figur 3 nicht gezeigt.

Die erfindungsgemäße stationäre Ethylenoxid-Sterilisationsanlage weist auch hinsichtlich des EO-Monitorings und der Sicherheit in Bezug auf EO-Kontamination deutliche Vorteile auf. Bei den herkömmlichen Sterilisationsanlagen musste ein EO-Monitoring in allen Gebäuderäumen durchgeführt werden, wo Bauteile der Sterilisationsanlage untergebracht waren. Bei der erfindungsgemäßen stationären Ethylenoxid-Sterilisationsanlage beschränkt sich die Ethylenoxid-Überwachung auf den Containerinnenraum und/oder die Abluft.

Durch die Containerbauweise verringert sich zudem das Raumvolumen und somit die Luftmenge für die notwendige Absaugung der Luft. Dies wiederum hat den Vorteil, dass die Abluftanlage kleiner dimensioniert werden kann.

### Figur 5

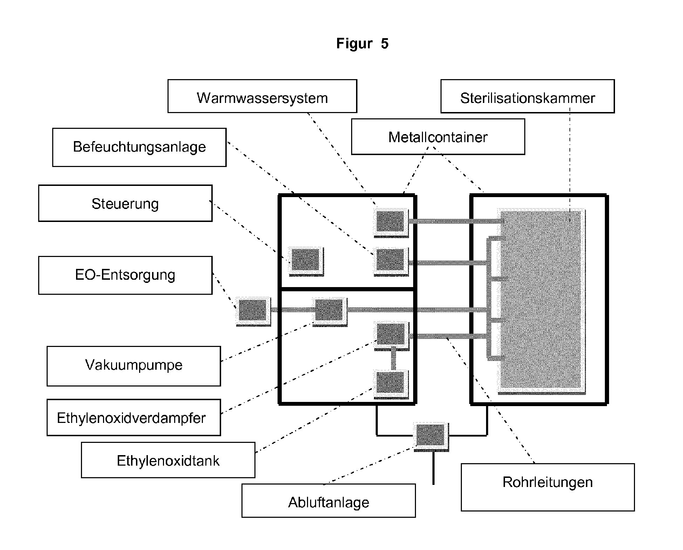

Figur 5 zeigt einen schematischen Aufbau einer erfindungsgemäßen stationären Ethylenoxid-Sterilisationsanlage umfassend eine Vakuumpumpe 5, einen Ethylenoxidverdampfer 4, einen Ethylenoxidtank 3, eine Entsorgungsanlage für das Ethylenoxid (EO-Entsorgung), welche sich außerhalb der Metallcontainer befindet, eine Steuerung oder Steuervorrichtung6, eine Befeuchtungsanlage 7 in Form eines Dampfgenerators 7, erforderliche Rohrleitungen 8 und eine Sterilisationskammer 2 sowie die außerhalb der Metallcontainer befindliche Abluftanlage 9. Die explosionsgefährdeten Bauteile umfassend die Sterilisationskammer 2, die Vakuumpumpe 5, den Ethylenoxidverdampfer 4 und den Ethylenoxidtank 3, wobei Vakuumpumpe 5, Ethylenoxidverdampfer 4 und Ethylenoxidtank 3 in einem ersten normierten Metallcontainer und die Sterilisationskammer 2 in einem damit verbundenen normierten zweiten Metallcontainer untergebracht sind. Auch innerhalb des ersten Metallcontainers aber in einem nicht zwingend explosionsgeschützten Bereich des ersten Metallcontainers sind ferner Befeuchtungsanlage 7, Steuerung oder Steuervorrichtung 6 sowie ein Warmwassersystem vorgesehen.

Die Rohrleitungen 8 zwischen den beiden normierten Metallcontainern beschränken sich auf Leistungen zwischen dem Warmwassersystem und der Sterilisationskammer 2, der Befeuchtungsanlage 7 und der Sterilisationskammer 2, der Vakuumpumpe 5 und der Sterilisationskammer 2 sowie dem Ethylenoxidverdampfer 4 und der Sterilisationskammer 2. Nahezu der vollständige Innenraum des normierten zweiten Metallcontainers wird für die Sterilisationskammer 2 zur Verfügung gestellt. Beide Metallcontainer und vorzugsweise Seecontainer werden bevorzugt in unmittelbarer Nähe zueinander aufgestellt und endmontiert. Die Abluftanlage 9 befindet sich zwangsläufig außerhalb der beiden Metallcontainer und ist mit den explosionsgeschützten Bereichen beider Metallcontainer verbunden.

### Referenzzeichenliste

- 1: Metallcontainer
- 2: Sterilisationskammer
- 3: Ethylenoxidtank oder Ethylenoxidflasche
- 4: Ethylenoxidverdampfer
- 5: Vakuumpumpe
- 6: Steuerung oder Steuervorrichtung
- 7: Befeuchtungsanlage bzw. Dampfgenerator
- 8: Rohrleitungen
- 9: Abluftanlage
- 10: Ethylenoxid(EO)-Sensor

### Figurenbeschreibung

### Figur 1

Figur 1 zeigt eine erfindungsgemäße stationäre Ethylenoxid-Sterilisationsanlage umfassend eine Vakuumpumpe, einen Ethylenoxidverdampfer, einen Ethylenoxidtank, eine Steuerung oder Steuervorrichtung, einen Dampfgenerator, eine Sterilisationskammer und erforderliche Rohrleitungen. Es ist zu erkennen, dass die explosionsgefährdeten Bauteile, nämlich Vakuumpumpe, Ethylenoxidverdampfer, Ethylenoxidtank und Sterilisationskammer der stationären Ethylenoxid-Sterilisationsanlage in einem Metallcontainer untergebracht sind und nur wenige Rohrleitungen durch die Containerwand führen. Die Steuerung oder Steuervorrichtung ist außen am Metallcontainer angebracht.

### Figur 2

Figur 2 zeigt die Anbindung einer erfindungsgemäßen stationären Ethylenoxid-Sterilisationsanlage an eine Abluftanlage.

### Figur 3

Figur 3 zeigt bevorzugte Stellen, wo EO-Sensoren in der Anbindung der erfindungsgemäßen stationären Ethylenoxid-Sterilisationsanlage an eine Abluftanlage vorgesehen werden können.

### Figur 4

Figur 4 zeigt eine erfindungsgemäße stationäre Ethylenoxid-Sterilisationsanlage umfassend eine Vakuumpumpe, einen Ethylenoxidverdampfer, einen Ethylenoxidtank, eine Steuerung oder Steuervorrichtung, einen Dampfgenerator, eine Sterilisationskammer und erforderliche Rohrleitungen. Es ist zu erkennen, dass die explosionsgefährdeten Bauteile, nämlich Vakuumpumpe, Ethylenoxidverdampfer, Ethylenoxidtank und Sterilisationskammer der stationären Ethylenoxid-Sterilisationsanlage in einem Metallcontainer untergebracht sind. Zudem sind in einem räumlich getrennten Bereich innerhalb des Metallcontainers die Steuerung oder Steuervorrichtung als auch der Dampfgenerator vorgesehen. Nur sehr wenige Rohrleitungen führen durch die Containerwand.

### Figur 5

Figur 5 zeigt eine erfindungsgemäße stationäre Ethylenoxid-Sterilisationsanlage, wobei in einem normierten ersten Metallcontainer in einem abgetrennten explosionsgeschützten Raum dieses ersten Metallcontainers Vakuumpumpe, Ethylenoxidverdampfer und Ethylenoxidtank vorgesehen sind und innerhalb dieses ersten Metallcontainers in einem davon abgetrennten Bereich Befeuchtungsanlage, Steuerung und Warmwassersystem untergebracht sind. In räumlicher Nähe zu diesem ersten Metallcontainer befindet sich ein normierter zweiter Metallcontainer, worin die Sterilisationskammer untergebracht ist.

### Figur 6

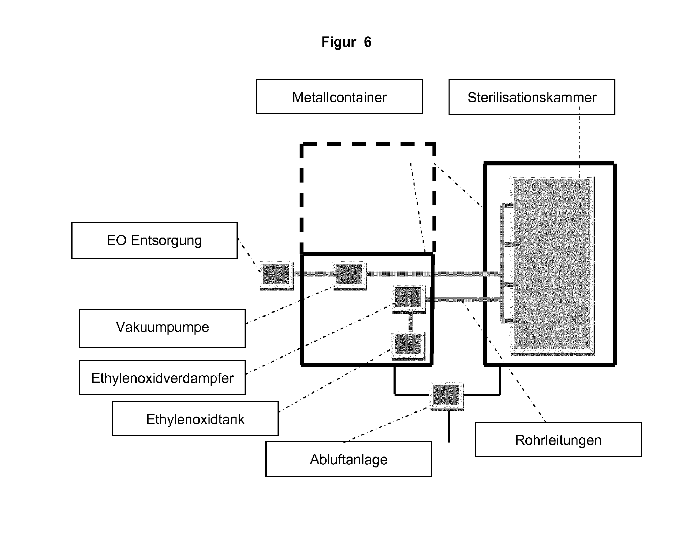

Figur 6 zeigt den Aufbau der stationären Ethylenoxid-Sterilisationsanlage gemäß Figur 5, wobei der nicht explosionsgeschützte Bereich des ersten Metallcontainers durch gestrichelte Linien markiert ist.

## Patentansprüche

1. Stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, **dadurch gekennzeichnet, dass** die Sterilisationskammer, die Vakuumpumpe, der Ethylenoxidverdampfer und der Ethylenoxidtank in mindestens einem Metallcontainer untergebracht sind.

2. Stationäre Ethylenoxid-Sterilisationsanlage gemäß Anspruch 1, wobei die Steuerung außerhalb des mindestens einen Metallcontainers oder von außen an dem mindestens einen Metallcontainer angebracht ist.

3. Stationäre Ethylenoxid-Sterilisationsanlage gemäß Anspruch 1, wobei die Steuerung in einem räumlich abgetrennten Bereich innerhalb des mindestens einen Metallcontainers angebracht ist.

4. Stationäre Ethylenoxid-Sterilisationsanlage gemäß Anspruch 1, 2 oder 3, wobei es sich bei dem mindestens einen Metallcontainer um einen Seecontainer handelt.

5. Stationäre Ethylenoxid-Sterilisationsanlage gemäß einem der Ansprüche 1 - 4, wobei die Vakuumpumpe, der Ethylenoxidverdampfer und der Ethylenoxidtank in einem ersten Metallcontainer und die Sterilisationskammer in einem zweiten Metallcontainer untergebracht sind.

6. Stationäre Ethylenoxid-Sterilisationsanlage gemäß einem der Ansprüche 1 - 5, des weiteren umfassend Rohrleitungen zwischen Sterilisationskammer und Vakuumpumpe, zwischen Sterilisationskammer und Ethylenoxidverdampfer, zwischen Ethylenoxidverdampfer und Ethylenoxidtank sowie zwischen Sterilisationskammer und Befeuchtungsanlage.

7. Stationäre Ethylenoxid-Sterilisationsanlage gemäß einem der Ansprüche 1 - 6, wobei die Befeuchtungsanlage und die Steuerung in einem räumlich abgetrennten Bereich innerhalb des mindestens einen Metallcontainers vorgesehen sind.

8. Stationäre Ethylenoxid-Sterilisationsanlage gemäß einem der Ansprüche 1 - 7, wobei sich die explosionsgefährdeten Bauteile innerhalb des mindestens einen Metallcontainers befinden.

9. Stationäre Ethylenoxid-Sterilisationsanlage des weiteren umfassend eine Abgasreinigungsanlage und eine Kammerheizung, wobei sich Abgasreinigungsanlage und Kammerheizung innerhalb des mindestens einen Metallcontainers befinden.

10. Stationäre Ethylenoxid-Sterilisationsanlage gemäß einem der Ansprüche 1 - 9 wobei es sich bei der stationären Ethylenoxid-Sterilisationsanlage um eine Palettensterilisationsanlage handelt.
